(19) Europäisches Patentamt
European Patent Office
Office européen des brevets

(11) **EP 3 407 079 B1**

(12) **EUROPEAN PATENT SPECIFICATION**

(45) Date of publication and mention
of the grant of the patent:
**07.12.2022 Bulletin 2022/49**

(21) Application number: **18163817.2**

(22) Date of filing: **25.03.2018**

(51) International Patent Classification (IPC):
*G01R 33/24* (2006.01)   *A61B 5/0536* (2021.01)
*A61B 5/055* (2006.01)   *G01R 33/48* (2006.01)
*G01R 33/483* (2006.01)   *A61B 18/00* (2006.01)

(52) Cooperative Patent Classification (CPC):
**G01R 33/243; A61B 5/0536; A61B 5/055;
G01R 33/4808; G01R 33/4833**

(54) **AN INDUCED CURRENT MAGNETIC RESONANCE ELECTRICAL IMPEDANCE TOMOGRAPHY (ICMREIT) PULSE SEQUENCE BASED ON MONOPOLAR SLICE SELECTION GRADIENT PULSES**

AUF MONOPOLAREN SCHICHTSELEKTIONSGRADIENTENIMPULSEN BASIERENDE PULSSEQUENZ FÜR INDUKTIONSSTROM-MAGNETRESONANZ-ELEKTROIMPEDANZ-TOMOGRAPHIE

SÉQUENCE D'IMPULSIONS DE TOMOGRAPHIE PAR IMPÉDANCE ÉLECTRIQUE À RÉSONANCE MAGNÉTIQUE ET À COURANT INDUIT (ICMREIT) BASÉE SUR DES IMPULSIONS MONOPOLAIRES DE GRADIENT DE SÉLECTION DE TRANCHES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.05.2017 TR 201707410**

(43) Date of publication of application:
**28.11.2018 Bulletin 2018/48**

(73) Proprietor: **ORTA DOGU TEKNIK UNIVERSITESI
06800 Ankara (TR)**

(72) Inventors:
• **EYUBOGLU, Behcet Murat**
 **06800 Ankara (TR)**
• **EROGLU, Hasan Huseyin**
 **06800 Ankara (TR)**
• **SADIGHI, Mehdi**
 **06800 Ankara (TR)**

(74) Representative: **Yalçiner Patent and Consulting
Ltd.
Tunus Caddesi 85/3-4
Kavaklidere
Ankara (TR)**

(56) References cited:
• **EROGLU HASAN H ET AL: "Induced current magnetic resonance electrical impedance tomography with z-gradient coil", 2014 36TH ANNUAL INTERNATIONAL CONFERENCE OF THE IEEE ENGINEERING IN MEDICINE AND BIOLOGY SOCIETY, IEEE, 26 August 2014 (2014-08-26), pages 1143-1146, XP032675099, DOI: 10.1109/EMBC.2014.6943797 [retrieved on 2014-11-02]**
• **OMER FARUK ORAN ET AL: "Feasibility of conductivity imaging using subject eddy currents induced by switching of MRI gradients : Feasibility of Conductivity Imaging Using Switching of MRI Gradients", MAGNETIC RESONANCE IN MEDICINE., vol. 77, no. 5, 1 July 2016 (2016-07-01), pages 1926-1937, XP055528661, US ISSN: 0740-3194, DOI: 10.1002/mrm.26283**
• **STEFANO MANDIJA ET AL: "A geometrical shift results in erroneous appearance of low frequency tissue eddy current induced phase maps : Geometrical Shift and Errors in Phase Maps", MAGNETIC RESONANCE IN MEDICINE., vol. 76, no. 3, 21 September 2015 (2015-09-21), pages 905-912, XP055528687, US ISSN: 0740-3194, DOI: 10.1002/mrm.25981**

- **LEVENT ÖZPARLAK ET AL: "Induced current magnetic resonance-electrical impedance tomography; Induced current MR-EIT",** PHYSIOLOGICAL MEASUREMENT, INSTITUTE OF PHYSICS PUBLISHING, BRISTOL, GB, vol. 26, no. 2, 1 April 2005 (2005-04-01), pages S289-S305, XP020092169, ISSN: 0967-3334, DOI: 10.1088/0967-3334/26/2/027
- **HASAN H. EROGLU ET AL: "Induced Current Magnetic Resonance Electrical Conductivity Imaging With Oscillating Gradients",** IEEE TRANSACTIONS ON MEDICAL IMAGING., vol. 37, no. 7, 23 January 2018 (2018-01-23), pages 1606-1617, XP055528311, US ISSN: 0278-0062, DOI: 10.1109/TMI.2018.2795718
- **Hasan Hüseygn Eroglu: "INDUCED CURRENT MAGNETIC RESONANCE ELECTRICAL IMPEDANCE TOMOGRAPHY (ICMREIT) WITH LOW FREQUENCY SWITCHING OF GRADIENT FIELDS",** PhD thesis, 1 July 2017 (2017-07-01), pages 1-150, XP055529642, Graduate School of Natural and Applied Sciences, Middle East Technical University, Turkey Retrieved from the Internet: URL:http://etd.lib.metu.edu.tr/upload/1262 1104/index.pdf [retrieved on 2018-12-03]

## Description

## Technical Field

Subject of the present invention is related to Induced Current Magnetic Resonance Electrical Impedance Tomography (ICMREIT) Pulse Sequence Based on Monopolar Slice Selection Gradient Pulses that induces low frequency eddy current in biological tissues or conducting material of interest by using the slice selection gradient coil of a magnetic resonance imaging (MRI) scanner and accumulates induced eddy current related phase in magnetic resonance (MR) phase images.

## Background of the Invention (Prior Art)

Magnetic Resonance Electrical Impedance Tomography (MREIT) is a method which enables high-resolution imaging of electrical conductivity of biological tissues by means of applying electrical current to the tissue and utilizing MRI methods.

MREIT is disclosed in the patent numbered US6397095 B1 and titled "Magnetic resonance-electrical impedance tomography." In MREIT, electrical current is injected into the object or the tissue to be imaged through surface electrodes in synchrony with an MRI pulse sequence. The injected current distributes inside the object being imaged according to the conductivity distribution of the object and creates a magnetic flux density distribution throughout the object. The component of the magnetic flux density related to the injected current in the direction of the main magnetic field of the MRI scanner accumulates phase in MR images. This phase is measured by MR phase imaging method. The electric potential field created by the injected current is measured at the surface of the imaged object by means of electrodes. By using the MR phase images and the surface potential measurements, the electrical conductivity distribution of the object is reconstructed.

The MREIT method has been studied since the beginning of the 1990's and the method provides unique contrast information completely different than the conventional MR images. However, the method has not been used in clinical applications due to the necessity of applying the electrical current through surface electrodes, generation of high current density in the regions close to the electrodes, and the difficulty of probing the electrical current to the regions surrounded by highly resistive structures, such as the brain surrounded by the skull.

In order to overcome the application difficulties of MREIT, in the paper titled Induced Current Magnetic Resonance Electrical Impedance Tomography, Physiol. Meas., vol. 26, no. 2, pp. 289-305 (2005), Özparlak and İder suggest the ICMREIT method and evaluated the performance of the method through computer simulations. In ICMREIT, a coil is located near the object or the tissue to be imaged. This coil is excited with low frequency electrical current, in synchrony with an MRI pulse sequence which is commonly based on spin-echo. Due to the excitation, eddy current, which is 90° phase lagged the coil current, is induced in the object being imaged. The induced eddy current, related to the conductivity characteristics of the imaged object, creates a secondary magnetic flux density distribution throughout the object. By applying 180° radio frequency (RF) pulses at the zero crossings of the eddy current on the timescale axis, a phase component is added to the MR images and the phase component related to the eddy current is measured by means of the MR phase imaging method. By using the readily known primary magnetic flux density distribution created by the coil current and the secondary magnetic flux density distribution measured by the MR phase imaging method, the low frequency electrical conductivity distribution of the object is reconstructed.

Experimental implementation of the suggested method of this study has not been realized up to now, since the method necessitates an extra coil structure that should be added to the MRI scanners. The necessity of adding an extra coil structure to the MRI scanners in the method suggested by the subject study is different from the method suggested by this invention. The manner of application, the number, and the order of 180° RF pulses in this invention and the subject study differ from each other.

In order to implement ICMREIT with the readily available gradient coils of the MRI scanners, Özsüt recommends a spin-echo based pulse sequence in his Master of Science thesis titled Design and Implementation of Labview Based Data Acquisition and Image Reconstruction Environment for METU-MRI System, Graduate School of Natural and Applied Sciences, Department of Electrical and Electronic Engineering, ODTÜ, Ankara, Turkey (2005). In this sequence, the frequency encoding gradient coil is excited with a waveform including a positive and a negative triangular pulse during the interval between the 90° excitation and the 180° refocusing RF pulses of the conventional spin-echo MRI pulse sequence. In order to accumulate phase contribution of the induced object eddy current to the MR phase, 180° RF pulses are applied at the peak instants of the gradient waveform corresponding to the zero crossings of the eddy current on the timescale axis. In this study, k-space magnitude images are inspected and it is observed that the k-space signal changes depending on the amplitude of the applied gradient waveform. The conductivity image of the imaged object is not reconstructed by means of the method applied in the study. The gradient coil type, the order of use, the number, and the manner of application of the gradient waveform and the 180° RF pulses in this invention and the subject study differ from each other.

In order to implement ICMREIT experimentally, Mandija et al. suggest a conventional spin-echo based pulse sequence in the paper titled A Geometrical Shift Results in Erroneous Appearance of Low Frequency Tissue Eddy Induced Current Phase Maps, Magn. Res.

Med., vol. 76, no. 3, pp. 905-912, (2016). In this study, a frequency encoding gradient pulse is located symmetrically around the 180° refocusing RF pulse of the conventional spin-echo pulse sequence. It is aimed to induce the eddy current during the rise and the fall times of the frequency encoding gradient pulse and add the eddy current related phase to the MR phase images. The pulse sequence is applied twice with positive and negative eddy current inducing gradient polarities and by taking the difference of the resultant MR phase images, it is aimed to obtain the eddy current related MR phase. Since the eddy current inducing gradient consists of a single pulse, ICMREIT has not been realized by the suggested method. The pulse sequence used in the subject study does not include the use of multiple gradient and 180° RF pulses and resembles the conventional spin-echo pulse sequence. The type of the eddy current inducing gradient coil, the gradient waveform, the number and the manner of application of 180° RF pulses in the subject study and the present invention differ from each other.

In order to implement ICMREIT experimentally, Oran and ider suggest a conventional spin-echo based pulse sequence in the paper titled Feasibility of Conductivity Imaging Using Subject Eddy Currents Induced by Switching of MRI Gradients, Magn. Res., Med., vol. 77, no. 5, pp. 1926-1937, (2016). In this study, it is aimed to induce the eddy current during the rise and the fall times of the slice selection gradient pulse located symmetrically around the 180° refocusing RF pulse of the conventional spin-echo pulse sequence and add the eddy current related phase to the MR phase images. The pulse sequence is applied twice with positive and negative eddy current inducing gradient polarities and by taking the difference of the resultant MR phase images, it is aimed to obtain the eddy current related MR phase. Since the eddy current inducing gradient consists of a single pulse, ICMREIT has not been realized by the suggested method. The type of the eddy current inducing gradient waveform, the number and the manner of application of 180° RF pulses in the subject study and the present invention differ from each other.

In order to implement ICMREIT experimentally, Gibbs and Liu suggest a conventional gradient-echo based pulse sequence in the paper titled Feasibility of Imaging Tissue Electrical Conductivity by Switching Field Gradients with MRI, Tomography, vol. 1, no. 2, pp. 125-135, (2015). In this study, a spoiler pulse is applied to the frequency encoding gradient, before the gradient-echo pulse sequence. By keeping the duration of the spoiler pulse long, it is aimed to induce the eddy current in the object imaged during only the fall time of the pulse and adding the eddy current related phase to the MR phase images. The pulse sequence is applied twice with positive and negative eddy current inducing gradient polarities and by taking the difference of the resultant MR phase images, it is aimed to obtain the eddy current related MR phase. Since the inductance values of the imaged objects are negligible, the current induced before the gradient echo sequence vanishes; and for this reason, it is not possible to measure the eddy current related phase by the suggested method. The type of the eddy current inducing gradient coil, the gradient waveform and its manner of application in the subject study and the present invention differ from each other.

The MRI pulse sequence Gibbs and Liu suggested in their paper titled Feasibility of Imaging Tissue Electrical Conductivity by Switching Field Gradients with MRI, Tomography, vol. 1, no. 2, pp. 125-135, (2015) is used in the measurement of system eddy current distributions induced in the conductive structures of the MRI scanners and in the correction of the distortions created by the system eddy current. In the patent numbered US4978919 A  and titled "Measurement and calibration of eddy currents for magnetic resonance imagers," a pulse sequence is suggested for the measurement of system eddy current and correction of its effects. In this sequence, one of the gradient coils is excited with a pulse, and after the pulse, spins are excited by means of RF pulses. After the excitation, time variation of the phase of free induction decay signal is measured. By means of the measured curves demonstrating the time variation of the phase, the characteristics of the system eddy current are evaluated and measures are taken for the correction of the distortions related with the system eddy current. The measurement of the system eddy current addressed by the subject patent and the formation of a pulse sequence that induces low frequency electrical current in biological tissues addressed by the present invention are different from each other. The order of use, the number, and the manner of application of the gradient and the 180° RF pulses in the subject patent and the present invention differ from each other.

In the patent numbered WO 2013057655 A1 and titled "MR electrical properties tomography" present in the known state of art, the object whose electrical properties are to be imaged is excited by means of a pulse sequence including at least a single RF and gradient pulse. The pulse sequence is applied at least twice with opposite gradient polarities at each application and the RF electrical properties of the object are recovered by utilizing the measured phase images. While the subject patent aims to find out the RF electrical properties of the objects, the present invention aims to find out the low frequency electrical properties of the objects by means of gradient pulses. The order of use, the number and the manner of application of the gradient and the 180° RF pulses in the subject patent and the present invention differ from each other. In the patent application numbered P16/0494 and titled "Çift Kutuplu Gradyan Darbe Tabanli Akim İndüklemeli Manyetik Rezonans Elektriksel Empedans Tomografisi (AİMREET) Darbe Dizini" present in the known state of art, induction of the low frequency eddy current in the object to be imaged by exciting the MR gradient coils in synchrony with a spin-echo based pulse sequence and addition of object eddy current related phase to the MR images are provided. In this application, spins in the imaged slice are excited with a selective $\alpha°$ (or 90°) RF pulse. After the application of the slice selection gradient of the selective RF pulse, the phase and the frequency encoding gradients, x, y, or, z gradients of the MRI scanner or their binary or trinary versions are excited by means of a bipolar waveform. The waveform includes positive and negative gradient pulses. During the rise and the fall times of the gradient pulses, eddy current is induced in the object imaged. Since non-selective hard 180° RF pulses are applied on the centre of the gradient plateaus where the gradient pulse amplitudes remain constant, phase contribution of the eddy current induced during the rise and the fall times of the gradient pulses do not destroy each other. The sign of the phase created by the low frequency eddy current changes and it is added to the phase created by the next current pulse. After the final gradient pulse, the 180° refocusing RF pulse of the conventional spin echo pulse sequence is applied together with the slice selection gradient. The spin echo signal is acquired after a time period equal to the interval between the peak instants of the $\alpha°$ (or 90°) RF pulse and the final refocusing 180° RF pulse. In the patent application numbered P16/0494, applying the hard 180° RF pulses together with the positive and the negative gradient pulses reduces the imaging performance. Because when the hard 180° RF pulses are applied with the gradient pulses, they lose the ability to be non-selective and their effect on the spins in the imaged slice decreases. As a result, ring-type artefacts are encountered in the measured MR images. In the pulse sequence proposed in this patent application, the eddy current inducing gradient waveform is applied to the slice selection gradient in monopolar form, soft and hard 180° RF pulses are applied when the gradient is non-zero and zero, respectively to prevent ring artefacts and optimize the imaging performance. Moreover, in order to form the MR echo signal in the maximum amplitude, the echo signal is acquired at the time instant which can be obtained by adding the interval between the first and the last 180° RF pulses to double the interval between the $\alpha°$ (or 90°) RF pulse and the first 180° RF pulse applied with the gradient waveform, instead of double the interval between the peak instants of the $\alpha°$ (or 90°) RF pulse and the final 180° refocusing RF pulse. By this way, the loss in echo signal due to spin-lattice (T1) relaxation is reduced. Even though the patent numbered P16/0494 and the present patent application aim to add the phase component to the MR images by means of inducing the eddy current in the object, the order of use, the number, the manner of application of the RF and the gradient pulses in the pulse sequences used in order to achieve this goal differ from each other. In view of the present applications in the known state of art, it is observed that there is no application having the same properties as the subject an Induced Current Magnetic Resonance Electrical Impedance Tomography (ICMREIT) Pulse Sequence Based on Monopolar Slice Selection Gradient Pulses.

The articles,

H. H. Eroğlu and B. M. Eyüboğlu, "Induced current magnetic resonance electrical impedance tomography with z-gradient coils," in Proc. IEEE Int. Conf. Eng. in Med. and Biol. (EMBC 2014), Chicago, 2014, pp. 1143-1146.

Ö. F. Oran and Y. Z. İder, "Feasibility of conductivity imaging using subject eddy currents induced by switching of MRI gradients," Magn. Reson. Med., vol. 77, no. 5, pp. 1926-1937, 2017.

discloses systems comprising ICMREIT pulse sequence

List of

MRI: magnetic resonance imaging

MR: magnetic resonance

MREIT: Magnetic Resonance Electrical Impedance Tomography

RF: Radio frequency (RF)

T1: Spin-lattice relaxation time constant

ICMREIT: Induced Current Magnetic Resonance Electrical Impedance Tomography

RF-p: RF pulses
$S_x\alpha°$: Selective and soft $\alpha°$ RF pulse applied in the x direction
$S_y180°$: Selective and soft 180° RF pulse applied in the y direction
$H_y180°$: Non-selective and hard 180° RF pulse applied in the y direction
$t_a$: Time interval between the $S_x\alpha°$ pulse and the beginning of current inducing gradient pulses
$t_B$: Time interval between the $S_x\alpha°$ pulse and the $S_y180°$ pulse applied for the first time
$t_C$: Time interval between the $S_y180°$ pulse and the $H_y180°$ pulses
$t_D$: Time interval between the $S_y180°$ pulses applied for the first and the last time
$T_E$: Echo time
$SS\text{-}G_z^+$: Slice selection gradient waveform with positive polarity
$SS\text{-}G_z^+[S_x\alpha°]$: Slice selection gradient of the $S_x\alpha°$ pulse with positive polarity
$PhR\text{-}G_z^+[S_x\alpha°]$: Phase rewinder gradient of the slice selection gradient of the $S_x\alpha°$ pulse with positive polarity
$t_r$: Rise and fall times of current inducing gradient pulses
$IC\text{-}G_z^+$: Current inducing gradient pulse with positive polarity

$T_{plt}$: Plateau time of the current inducing gradient pulses

SS-$G_z^-$: Slice selection gradient waveform with negative polarity

SS-$G_z^-$[$S_x\alpha°$]: Slice selection gradient of the $S_x\alpha°$ pulse with negative polarity

PhR-$G_z^-$[$S_x\alpha°$]: Phase rewinder gradient of the slice selection gradient of the $S_x\alpha°$ pulse with negative polarity

IC-$G_z^-$: Current inducing gradient pulse with negative polarity

$J_{LF}^+$: Low frequency (LF) eddy current induced by SS-$G_z^+$

$J_{LF}^-$: LF eddy current induced bv SS-$G_z^-$

PhE-$G_y$:Phase encoding gradient

$G_y$-p: $G_y$ pulse

FrE-$G_x$: Frequency encoding gradient

$G_x$-p: $G_x$ pulse

DAQ-$G_x$:Data acquisition part of the $G_x$ pulse

Loc: Localizer slice

PhySlc: Physical slice

SymSlc: Symmetrical slice selected on the operator console of the MR scanner

**Brief Description and the Purpose of the Invention**

The present invention is defined by independent claim 1 and is related to an Induced Current Magnetic Resonance Electrical

Impedance Tomography (ICMREIT) Pulse Sequence Based on Monopolar Slice Selection Gradient Pulses and the invention is an MRI pulse sequence that provides the optimized realization of ICMREIT by exciting the MRI slice selection gradient coil with a monopolar waveform and application of the selective soft 180° RF pulses with the gradient waveform.

The primary advantage of the present invention is the realization of an MR based low frequency conductivity imaging method having the possibility of clinical application by minimizing the artefacts resulting from the non-ideal RF and gradient pulses.

The purpose of the present invention is to realize ICMREIT by adding low frequency phase only to the spins in the imaged slice by utilizing the MRI slice selection gradient coil. Another purpose of the present invention is to implement ICMREIT without the necessity to add extra hardware to the MRI scanner.

Another purpose of the present invention is to use a monopolar slice selection gradient waveform in order to induce the eddy current in ICMREIT. Another purpose of the present invention is to use slice selective soft 180°RF pulses with the slice selection gradient waveform used in ICMREIT. By this way, it is aimed to eliminate the off-slice effects resulted from the use of the gradient and the hard RF pulses together and minimize the possible ring type artefacts. According to the present invention, the $\alpha°$ (or 90°) RF pulse is applied along the x axis and the 180° RF pulses are applied along the y axis.

According to the present invention, the slice selection gradient waveform has either a positive or a negative polarity. According to the present invention, the slice to be imaged is chosen with respect to the localizer of the MR scanner from the operator console, if the slice selection gradient waveform has positive polarity, and is chosen symmetrically with respect to the localizer, if the slice selection gradient waveform has negative polarity, such that the same physical slice is selected regardless of whether the slice selection gradient waveform has positive or negative polarity.

According to the present invention, the amplitude of the slice selection gradient waveform is uniform and determined according to the slice thickness of the scanner.

According to the present invention, the slice selection gradient waveform consists of positive or negative trapezoidal gradient pulses. According to the present invention, slice selective soft 180° RF pulses, which enable the accumulation of the phase component related to the induced eddy current, are applied at the centre points of the slice selection gradient waveforms.

According to the present invention, the time interval between the peak points of the $\alpha°$ RF (or 90°) pulse and the first slice selective soft 180° RF pulse is equal to the time interval between the last slice selective soft 180° RF pulse and the centre point of the readout gradient.

According to the present invention, an application of non-slice selective hard 180° RF pulses provides the accumulation of the phase component related to the eddy current induced after the slice selective soft 180° RF pulse and the slice selection gradient pulse couple. According to the present invention, the time intervals between the successive slice selective soft 180° RF pulses and the non-slice selective hard 180° RF pulses are equal and the total number of the 180° RF pulses is odd.

According to the present invention, current inducing gradient pulses (IC-$G_z^+$) with positive polarity are applied together with only the selective soft 180° RF pulses ($S_y$180°) in the y direction when SS-Gz$^+$ is used, and current inducing gradient pulses (IC-$G_z^-$) with negative polarity are applied together with only the selective soft 180° RF pulses ($S_y$180°) in the y direction when SS-Gz$^-$ is used.

According to the present invention, the amplitudes and the polarities of the IC-$G_z^+$ and IC-$G_z^-$ pulses are equal to SS-$G_z^+$[$S_x\alpha°$] and SS-$G_z^-$[$S_x\alpha°$] respectively.

According to the present invention, the time interval between the $S_x\alpha°$ pulse and the first $S_y$180° pulse is equal to the time interval between the $S_y$180° pulse and the instant of the formation of a spin-echo signal. According to the present invention, the time intervals between all of the $S_y$180° and $H_y$180° pulses in the pulse sequence are equal to $t_C$, which is kept constant in order to satisfy the phase coherence of the spins.

According to the present invention, the total

number of $S_y180°$ and $H_y180°$ pulses in the pulse sequence is odd in order to obtain a measurable echo signal.

## Description of the Illustrative Figures

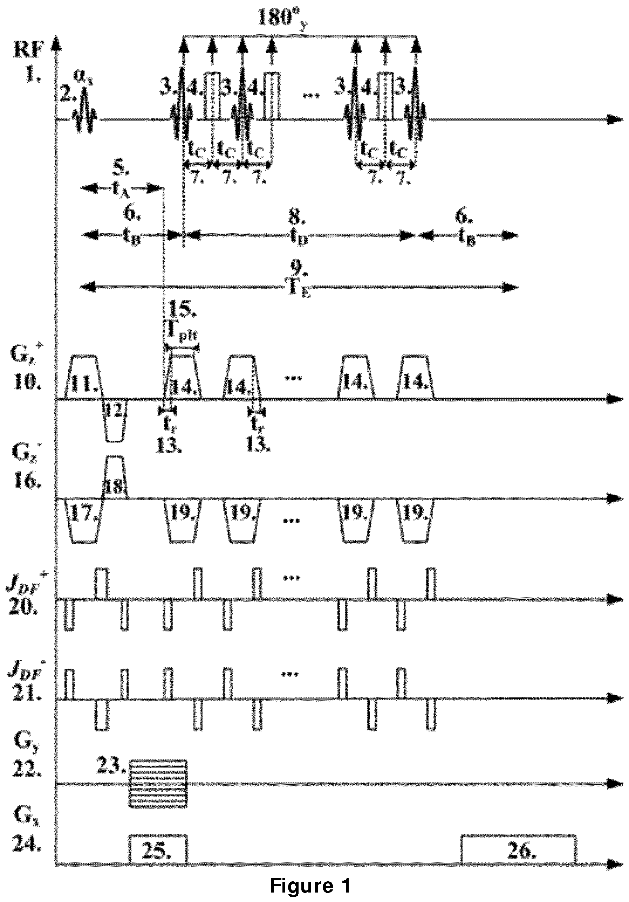

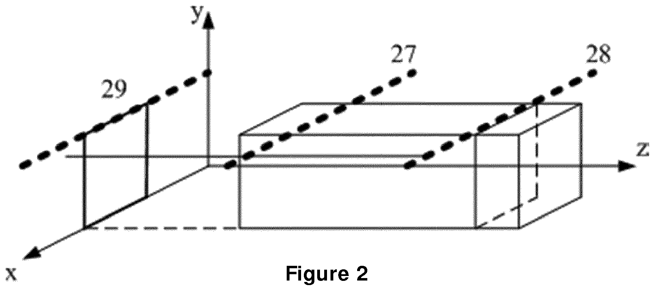

In order to better illustrate an Induced Current Magnetic Resonance Electrical Impedance Tomography (ICMREIT) Pulse Sequence Based on Monopolar Slice Selection Gradient Pulses, which has been developed by means of this invention, the figures prepared are further explained in the following. Figure 1- An Induced Current Magnetic Resonance Electrical Impedance Tomography (ICMREIT) Pulse Sequence Based on Monopolar Slice Selection Gradient Pulses
Figure 2- Slice Selection for an Induced Current Magnetic Resonance Electrical Impedance Tomography (ICMREIT) Pulse Sequence Based on Monopolar Slice Selection Gradient Pulses

## Description of the Elements/Sections/Parts That Constitute the Invention

In order to better explain An Induced Current Magnetic Resonance Electrical Impedance Tomography (ICMREIT) Pulse Sequence Based on Monopolar Slice Selection Gradient Pulses that has been developed with this invention, the elements/sections/parts in the prepared figures have been separately numbered and the explanation of each number is given below.

1. RF pulses (RF-p)
2. Selective and soft $\alpha°$ RF pulse ($S_x\alpha°$) applied in the x direction
3. Selective and soft 180° RF pulse ($S_y180°$) applied in the y direction
4. Non-selective and hard 180° RF pulse ($H_y180°$) applied in the y direction
5. The time interval ($t_A$) between the $S_x\alpha°$ pulse and the beginning of the current inducing gradient
6. The time interval ($t_B$) between the $S_x\alpha°$ pulse and the $S_y180°$ pulse applied for the first time
7. The time interval ($t_C$) between the $S_y180°$ pulse and the $H_y180°$ pulses
8. The time interval ($t_D$) between the $S_y180°$ pulses applied for the first and the last time
9. Echo time (TE)
10. Slice selection gradient with positive polarity (SS-$G_z^+$)
11. Slice selection gradient of the $S_x\alpha°$ pulse with positive polarity (SS-$G_z^+$[$S_x\alpha°$])
12. Phase rewinder gradient of the slice selection gradient of the $S_x\alpha°$ pulse with positive polarity (PhR-$G_z^+$[$S_x\alpha°$])
13. Rise and fall times of the current inducing gradient ($t_r$)
14. Current inducing gradient pulse with positive polarity (IC-$G_z^+$)

15. Plateau time of the current inducing gradient ($T_{plt}$)
16. Slice selection gradient with negative polarity (SS-$G_z^-$)
17. Slice selection gradient of the $S_x\alpha°$ pulse with negative polarity (SS-$G_z^-$[$S_x\alpha°$])
18. Phase rewinder gradient of the slice selection gradient of the $Y_x\alpha°$ pulse with negative polarity (PhR-$G_z^-$[$S_x\alpha°$])
19. Current inducing gradient pulse with negative polarity (IC-$G_z^-$)
20. Eddy current induced by SS-$G_z^+$ ($J_{LF}^+$)
21. Eddy current induced by SS-$G_z^-$ ($J_{LF}^-$)
22. Phase encoding gradient (PhE-$G_y$)
23. $G_y$ pulse ($G_y$-p)
24. Frequency encoding gradient (FrE-$G_x$)
25. $G_x$ pulse ($G_x$-p)
26. Data acquisition part of the $G_x$ pulse (DAQ-$G_x$)
27. Localizer slice (Loc)
28. Physical slice (PhySlc)
29. Symmetrical slice selected on the operator console of the MR scanner (SymSlc)

## Detailed Description of the Invention

In this detailed description, the subject innovation is only described through examples which will not create any limiting effect on the way to better understand the subject. Accordingly, elements constituting an Induced Current Magnetic Resonance Electrical Impedance Tomography (ICMREIT) Pulse Sequence Based on Monopolar Slice Selection Gradient Pulses are explained.

An Induced Current Magnetic Resonance Electrical Impedance Tomography (ICMREIT) Pulse Sequence Based on Monopolar Slice Selection Gradient Pulses is a pulse sequence which induces current in the biological tissues by using the MR slice selection gradient coil and accumulates the low frequency phase related to the selected slice and the induced current to the MR images. An Induced Current Magnetic Resonance Electrical Impedance Tomography (ICMREIT) Pulse Sequence Based on Monopolar Slice Selection Gradient Pulses is characterized in that it comprises the elements such as RF pulses (RF-p) (1), slice selection gradients with positive(SS-$G_z^+$) (10) or negative (SS-$G_z^-$) (16) polarities, phase encoding gradient (PhE-$G_y$)(22), and frequency encoding gradient FrE-$G_x$ (24).

An Induced Current Magnetic Resonance Electrical Impedance Tomography (ICMREIT) Pulse Sequence Based on Monopolar Slice Selection Gradient Pulses is characterized in that it comprises the procedure steps of:

• Application of $S_x\alpha°$ (2) in the x direction,
• Selection of PhySlc (28) on the operator console of the MR scanner when SS-$G_z^+$(10) is used,
• Application of SS-$G_z^+$[$S_x\alpha°$] (11) in order to excite the spins only in the PhySlc (28) and tip these spins into the transverse plane when SS-$G_z^+$ (10) is used,

- Determination of the amplitude of SS-$G_z^+[S_x\alpha°]$ (11) according to the slice thickness,
- Presence of PhySlc (28) on xy plane,
- Application of PhR-$G_z^+[S_x\alpha°]$ (12) in order to reduce the phase effect that will be created by SS-$G_z^+[S_x\alpha°]$ (11),
- Selection of SymSlc (29) on the operator console of the MR scanner when SS-$G_z^-$ (16) is used,
- Presence of SymSlc (29) on xy plane,
- Equal distance of PhySlc (28) and SymSlc (29) to Loc (27) along the z axis,
- When SS-$G_z^-$ (16) is used, selection of SymSlc (29) on the operator console of the MR scanner and providing the selection of PhySlc (28) in the physical condition,
- Application of SS-$G_z^-[S_x\alpha°]$ (17) in order to excite the spins only in the PhySlc (28) and tip these spins into the transverse plane when SS-$G_z^-$ (16) is used,
- Equality of the amplitude of SS-$G_z^-[S_x\alpha°]$ (17) to the amplitude of SS-$G_z^+[S_x\alpha°]$ (11)
- Opposite polarity of SS-$G_z^-[S_x\alpha°]$ (17) with respect to the polarity of SS-$G_z^+[S_x\alpha°]$ (11),
- Application of PhR-$G_z^-[S_x\alpha°]$ (18) in order to reduce the phase effect that will be created by SS-$G_z^-[S_x\alpha°]$ (17),
- Application of $G_y$-p (23) and $G_x$-p (25), simultaneously, for phase encoding, when SS-$G_z^+$ (10) or SS-$G_z^-$(16) are used,
- Application of IC-$G_z^+$ (14) in order to induce $J_{LF}^+$(20) in the imaged object when SS-$G_z^+$(10) is used,
- Application of IC-$G_z^-$ (19) in order to induce $J_{LF}^-$ (21) in the imaged object when SS-$G_z^-$(16) is used,
- The time interval between $S_x\alpha°$ (2) pulse and the first IC-$G_z^+$ (14) or IC-$G_z^-$ (19) pulses being $t_A$ (5),
- Rise and fall times of IC-$G_z^+$ (14) or IC-$G_z^-$ (19) pulses being $t_r$ (13),
- Plateau time of IC-$G_z^+$ (14) or IC-$G_z^-$ (19) pulses being $T_{plt}$ (15),
- Selection of the amplitudes of IC-$G_z^+$ (14) or IC-$G_z^-$ (19) pulses equal to the amplitudes of SS-$G_z^+[S_x\alpha°]$ (11) and SS-$G_z^-[S_x\alpha°]$ (17), respectively,
- Selection of the polarities of IC-$G_z^+$ (14) or IC-$G_z^-$ (19) pulses equal to the polarities of SS-$G_z^+[S_x\alpha°]$ (11) and SS-$G_z^-[S_x\alpha°]$ (17), respectively,
- Induction of $J_{LF}^+$(20) and $J_{LF}^-$ (21) on the imaged object during the $t_r$ (13) durations of IC-$G_z^+$ (14) or IC-$G_z^-$ (19) pulses, respectively,
- Application of $S_y180°$ (3) pulses in the y direction at the centre points of $T_{plt}$ (15) durations of IC-$G_z^+$(14) and IC-$G_z^-$ (15) in order to add the phase component related to $J_{LF}^+$(20) or $J_{LF}^-$ (21) to MR images,
- Time interval between the $S_x\alpha°$ (2) pulse and the first $S_y180°$ pulse (3) being $t_B$ (6),
- Application of $H_y180°$ (4) pulses after a time interval of $t_C$ (7) following $S_y180°$ (3) pulses in order to add the phase component related to $J_{LF}^+$(20) or $J_{LF}^-$ (21) to the MR images,
- Keeping the time interval of $t_C$ (7) between all $S_y180°$ (3) and $H_y180°$ (4) constant in order to preserve the phase coherence of spins,
- The time interval between the first and the last $S_y180°$ (3) pulses being $t_D$ (7),
- Total number of the $S_y180°$ (3) pulses and the $H_y180°$ (4) pulses being odd,
- Application of DAQ-$G_x$ (26) after a time interval of $t_B$ (6) following the last $S_y180°$ (3) pulse,
- Acquisition of the spin-echo signal symmetrically with respect to the instant of $T_E$ (9) and $T_E$ (9) being equal to the sum of twice the duration of $t_B$ (6) and the duration of $t_D$ (7),
- Obtaining two different MR phase images by using SS-$G_z^+$(10) or SS-$G_z^-$(16) in two different scans, naming the obtained MR phase images as $\Phi^+$ and $\Phi^-$, respectively,
- Obtaining the low frequency (LF) phase component ($\phi_{LF}$) added to the MR signal by taking the difference between $\Phi^+$ and $\Phi^-$.

An Induced Current Magnetic Resonance Electrical Impedance Tomography (ICMREIT) Pulse Sequence Based on Monopolar Slice Selection Gradient Pulses is a pulse sequence that induces current in the biological tissues using the MR slice selection gradient coil and adds the low frequency phase component related to the selected slice and the induced current to the MR images. The sequence consists of the elements of RF-p (1), SS-$G_z^+$(10) or SS-$G_z^-$(16), PhE-$G_y$ (22), and FRE-$G_X$ (24). In the sequence, first, the slice whose conductivity is required to be imaged is selected. When SS-$G_z^+$(10) is used, PhySlc (28) is selected on the operator console of the MR scanner. When SS-$G_z^-$(16) is used, SymSlc (29) is selected on the operator console of the MR scanner. PhySlc (28) and SymSlc (29) are on the xy plane and the distance of PhySlc (28) and SymSlc (29) to Loc (27) is equal along the z axis. By selecting the polarities of SS-$G_z^+$(10) and SS-$G_z^-$' (16) opposite with respect to each other and selecting their amplitudes equal, in both cases, it is provided that PhySlc (28) is physically selected. After selecting the slice required to be imaged, spins are selectively excited by $S_x\alpha°$ (2) RF pulse along the x axis. When SS-$G_z^+$ (10) is used, in order to provide the selective excitation of the slice required to be imaged with $S_x\alpha°$ (2) RF pulse, SS-$G_z^+[S_x\alpha°]$ (11) is applied simultaneously. Amplitude of SS-$G_z^+[S_x\alpha°]$ (11) is determined according to the slice thickness. In order to reduce the phase effect that will be created by SS-$G_z^+[S_x\alpha°]$ (11), PhR-$G_z^+[S_x\alpha°]$ (12) is applied. When SS-$G_z^-$ (16) is used, in order to provide the selective excitation of the slice required to be imaged with $S_x\alpha°$ (2) RF pulse, SS-$G_z^-[S_x\alpha°]$ (17) is applied simultaneously. Amplitude of SS-$G_z^-[S_x\alpha°]$ (17) is determined according to the slice thickness. In order to reduce the phase effect that will be created by SS-$G_z^-[S_x\alpha°]$ (17), PhR-$G_z^-[S_x\alpha°]$ (18) is applied. Immediately after the PhR-

$G_z^+[S_x\alpha°]$ (12) and PhR-$G_z^-[S_x\alpha°]$ (18) pulses, $G_y$-p (23) and $G_x$-p (25) pulses are applied for phase encoding. The application manner of the $S_x\alpha°$ (2), SS-$G_z^+[S_x\alpha°]$ (11), PhR-$G_z^+[S_x\alpha°]$ (12), SS-$G_z^-[S_x\alpha°]$ (17), PhR-$G_z^-[S_x\alpha°]$ (18), $G_y$-p (23), and $G_x$-p (25) pulses expressed up until this section has the same manner of application of the subject pulses in the conventional spin-echo pulse sequence. In order to induce $J_{LF}^+$ (20) or $J_{LF}^-$ (21) in the object imaged, IC-$G_z^+$ (14) or IC-$G_z^-$ (19) pulses are applied after a time interval of $t_A$ (5) following the $S_x\alpha°$ (2) pulse. During the $t_r$ (13) intervals of of IC-$G_z^+$ (14) and IC-$G_z^-$ (19) pulses, $J_{LF}^+$ (20) or $J_{LF}^-$ (21) are induced in the object to be imaged, respectively. Amplitudes and polarities of the IC-$G_z^+$ (14) or IC-$G_z^-$ (19) pulses are equal to SS-$G_z^+[S_x\alpha°]$ (11) and SS-$G_z^-[S_x\alpha°]$ (17), respectively. In order to add the phase component related to $J_{LF}^+$ (20) or $J_{LF}^-$ (21) to MR images, $S_y$180° (3) pulses are applied in the y direction, at the centre points of $T_{plt}$ (15) intervals of IC-$G_z^+$ (14) and IC-$G_z^-$ (15). The time interval between the $S_x\alpha°$ (2) and the first $Y_y$180° (3) pulses is $t_B$ (6). In order to add the phase component related to $J_{LF}^+$ (20) or $J_{LF}^-$ (21) to the MR images, $H_y$180° (4) pulses are applied after an interval of $t_C$ (7) following the $S_y$180° (3) pulses. The time interval between all $S_y$180° (3) and $H_y$180° (4) pulses in the pulse sequence is kept constant as $t_C$ (7); thus, the phase coherence of the spins is preserved. The time interval between the first and the last $S_y$180° (3) pulses is $t_D$ (7). In order to obtain a measurable echo signal, the total number of the $S_y$180° (3) and $H_y$180° (4) pulses in the pulse sequence is odd. After an interval of $t_B$ (6) following the last $S_y$180° (3) pulse, DAQ-$G_X$ (26) is applied symmetrically with respect to the $T_E$ (9) instant and the spin-echo signal is measured. The relationship between $T_E$ (9), $t_B$ (6), and $t_D$ (7) can be expressed as:

$$T_E = 2t_B + t_D.$$

By using SS-$G_z^+$ (10) or SS-$G_z^-$ (16) in two different scans, two different MR phase images are obtained, which can be named as $\Phi^+$ and $\Phi^-$, respectively. The phase component ($\Phi_{RF}$) created by the RF pulses in the $\Phi^+$ and the ($\Phi^-$ images is the same, wheras the low frequency phase component ($\Phi_{LF}$) induced by $J_{LF}^+$ (20) or $J_{LF}^-$ (21) have opposite polarities. The relationship between $\Phi^+$, $\Phi^-$, $\Phi_{RF}$ and $\Phi_{LF}$ can be expressed as

$$\Phi^+ = \Phi_{RF} + \Phi_{LF}$$

and

$$\Phi^- = \Phi_{RF} - \Phi_{LF}.$$

$\Phi_{LF}$ is calculated by using

$$\Phi_{LF} = \frac{\Phi^+ + \Phi^-}{2}.$$

X and y components of $J_{LF}^+$ (20) ($J_x$ and $J_y$) are obtained by using

$$J_x = \frac{\partial \Phi_{LF}}{\partial y} \text{ and } J_y = -\frac{\partial \Phi_{LF}}{\partial x}$$

The primary magnetic flux density ($B_p$) value of the slice selection gradient coil at PhySlc (28) is calculated by using the amplitude ($G_z$) of SS-$G_z^+[S_x\alpha°]$ (11) and the distance ($d_z$) between PhySlc (28) and Loc (27) along the z axis as:

$$B_p = G_z d_z.$$

The conductivity distribution ($\sigma$) inside PhySlc (28) is reconstructed by using

$$\sigma = \left(\frac{\partial J_y}{\partial x} - \frac{\partial J_x}{\partial y}\right)/\omega B_p,$$

where

$$\omega = 2\pi/(4t_r)$$

is the approximate angular frequency of SS-$G_z^+$ (10).
The performance of an Induced Current Magnetic Resonance Electrical Impedance Tomography (ICMREIT) Pulse Sequence Based on Monopolar Slice Selection Gradient Pulses has been tested with experimental phantoms. In the suggested method, adding an extra equipment to the MRI scanner is not required. This is one of the primary practical advantages of the suggested method in comparison with the conventional Magnetic Resonance Electrical Impedance Tomography (MREIT). In the suggested method, the use of the current inducing gradient pulses only with the soft pulses provides the minimization of the ring type artefacts resulting from the gradient pulse and the hard RF pulses being used together. The scan time of the proposed pulse sequence is similar to the scan period of the conventional spin-echo pulse sequence. For a repetition time of 500 ms, an averaging number of 4, and a reconstruction matrix size of 128x128 pixels, the scan time of the pulse sequence is 8 minutes. This time period is the same with the scan time of the spin echo sequence applied with the same values. The reconstruction time of the conductivity images by making use of the obtained phase images is between 2.5-10 seconds.
The application method of an Induced Current Magnetic Resonance Electrical Impedance Tomography (ICMREIT) Pulse Sequence Based on Monopolar Slice Selection Gradient Pulses is;

- Inducing the current in the object to be imaged by means of SS-G$_z$$^+$(10) or SS-G$_z$$^-$(16),
- Selecting the PhySlc (28) on the operator console of the MR scanner when SS-G$_z$$^+$(10) is used in the sequence,
- Selecting the SymSlc (29) on the operator console of the MR scanner when SS-G$_z$$^-$(16) is used in the sequence,
- PhySlc (28) and SymSlc (29) being on the xy plane,
- Distance of PhySlc (28) and SymSlc (29) to Loc (27) being equal along the z axis,
- By selecting the polarities of SS-G$_z$$^+$(10) and SS-G$_z$$^-$(16) opposite with respect to each other and selecting their amplitudes equal, providing PhySlc (28) is physically selected in both cases.

The application method of an Induced Current Magnetic Resonance Electrical Impedance Tomography (ICMREIT) Pulse Sequence Based on Monopolar Slice Selection Gradient Pulses according to the invention is defined by independent claim 1.

**Claims**

1. An application method of an Induced Current Magnetic Resonance Electrical Impedance Tomography (ICMREIT) Pulse Sequence Based on Monopolar Slice Selection Gradient Pulses applied by means of a slice selection gradient coil where two separate scans of a slice required to be imaged are performed, wherein a MR phase image is obtained in each of these scans by means of an application of a pulse sequence and wherein the two obtained MR phase images are different, wherein

    • a current is induced in an object to be imaged in said two separate scans by means of a slice selection gradient waveform with positive polarity, SS-G$_z$$^+$ in the first scan, and a slice selection gradient waveform with negative polarity, SS-G$_z$$^-$ in the second scan, said SS-G$_z$$^+$ or SS-G$_z$$^-$ slice selection gradient waveforms consisting of trapezoidal gradient pulses having equal amplitudes and opposite polarities with respect to each other,
    • wherein the slice required to be imaged is selected on the operator console of the MR scanner with respect to a Localizer slice, Loc, as
    • Physical slice, PhySlc, when SS-G$_z$$^+$is used in the pulse sequence, and as
    • Symmetrical slice, SymSlc when SS-G$_z$$^-$is used in the pulse sequence, such that SymSlc is chosen symmetrically to PhySlc with respect to Loc,
    • said PhySlc and SymSlc being in xy plane and the distance of PhySlc and SymSlc to Loc being equal along z axis, such that in both cases the slice required to be imaged corresponds to PhySlc, wherein the method comprises procedure steps of;
    • an excitation of spins with a selective and soft radio-frequency (RF) pulse S$_x$α°, applied along x axis after the slice required to be imaged is selected on the operator console of the MR scanner,
    • When SS-G$_z$$^+$ is used, an application of a positive polarity slice selection gradient of the S$_x$α° pulse, SS-G$_z$$^+$[S$_x$α°], simultaneously with S$_x$α° in order to provide a selective excitation of the slice required to be imaged with the S$_x$α° RF pulse, wherein the amplitude of SS-G$_z$$^+$[S$_x$α°] is determined according to slice thickness, followed by an application of a phase rewinder gradient PhR-G$_z$$^+$[S$_x$α°] in order to reduce phase effect created by SS-G$_z$$^+$[S$_x$α°],
    • When SS-G$_z$$^-$ is used, an application of a negative polarity slice selection gradient of the S$_x$α° pulse, SS-G$_z$$^-$[S$_x$α°] simultaneously with S$_x$α° in order to provide the selective excitation of the slice required to be imaged with the S$_x$α° RF pulse,

wherein
the amplitude of SS-G$_z$$^-$[S$_x$α°] is determined according to slice thickness, Followed by an application of a phase rewinder gradient PhR-G$_z$$^-$[S$_x$α°] in order to reduce phase effect created by SS-G$_z$$^-$[S$_x$α°],

    • Following the application of slice selection and phase rewinder gradient pulses, an application of phase encoding gradient pulses along x-axis, G$_x$-p, and along y-axis, G$_y$-p, immediately after the PhR-G$_z$$^+$[S$_x$α°] or PhR-G$_z$$^-$[S$_x$α°] pulses,
    • Following the application of the G$_y$-p and G$_x$-p pulses, an application of current inducing gradient pulses with positive polarity along z-axis, IC-G$_z$$^+$, in order to induce low frequency (LF) eddy current J$_{LF}$$^+$ in

the imaged object when SS-G$_z$$^+$ is used and an application of current inducing gradient pulses with negative polarity along z-axis, IC-G$_z$$^-$, in order to induce LF eddy current J$_{LF}$$^-$ in the imaged object when SS-G$_z$$^-$ is used, wherein the interval between the S$_x$α° pulse and the centre point of the plateau interval of the respective first IC-G$_z$$^+$ or IC-G$_z$$^-$ pulse is t$_B$, and wherein the interval between two adjacent IC-G$_z$$^+$ or IC-G$_z$$^-$ pulses is 2*t$_C$,
wherein the polarities and the amplitudes of said IC-G$_z$$^+$ or IC-G$_z$$^-$ pulses are equal to the polarities and the amplitudes SS-G$_z$$^+$[S$_x$α°] and SS-G$_z$$^-$[S$_x$α°] respectively,

• an application of selective and soft 180° pulses in y-direction, $S_y180°$, the centre of said pulses coinciding with the centre points of the plateau intervals of the IC-$G_z^+$ and IC-$G_z^-$ pulses in order to add a phase component related to $J_{LF}^+$ or $J_{LF}^-$ to the MR images,

• Following each $S_y180°$ pulse with the exception of the last $S_y180°$ pulse, an application of non-selective and hard 180° pulses in y-direction, $H_y180°$, after interval of $t_C$ following each of the $S_y180°$ pulses, in order to add a phase component related to $J_{LF}^+$ or $J_{LF}^-$ to the MR images, wherein the total number of $S_y180°$ and $H_y180°$ pulses in the pulse sequence is odd in order to obtain a measurable echo signal,

• Following the aforementioned pulses, a measurement of spin-echo signal formed at time interval $t_B$ after the last $S_y180°$ pulse, with the simultaneous application of a data acquisition gradient along x-axis symmetrically with respect to the spin-echo formation time,

• Obtaining said two different MR phase images by using SS-$G_z^+$ or SS-$G_z^-$ in said two different scans, from the respective measurements of the spin-echo signal,

• Naming said MR phase images obtained in scans with SS-$G_z^+$ or SS-$G_z^-$ as $\Phi^+$ and $\Phi^-$ respectively,

• Obtaining low frequency MR phase distribution $\Phi_{LF}$ as

$$\Phi_{LF} = \frac{\Phi^+ + \Phi^-}{2}$$

• Obtaining conductivity distribution $\sigma$ inside PhySlc as

$$\sigma = \frac{\left(\frac{\partial J_y}{\partial x} - \frac{\partial J_x}{\partial y}\right)}{\omega B_p},$$

Wherein Jx and Jy are obtained as

$$J_x = \frac{\partial \Phi_{LF}}{\partial y} \text{ and } J_y = -\frac{\partial \Phi_{LF}}{\partial x}$$

Wherein $B_p$ is the primary magnetic flux density value of the slice selection gradient coil at PhySlc and is calculated by using amplitude $G_z$ of SS-$G_z^+[S_x\alpha°]$ or SS-$G_z^-[S_x\alpha°]$ and distance $d_z$ between PhysSlc and Loc along the z axis as:

$$B_p = G_z d_z$$

wherein $\omega$ is obtained as

$$\omega = 2\pi/(4t_r)$$

wherein $t_r$ corresponds to rise and fall times of IC$^-$Gz$^+$ or IC$^-$Gz$^-$ pulses.

**Patentansprüche**

1. Verfahren zur Anwendung einer Pulssequenz für Induktionsstrom-Magnetresonanz-Elektroimpedanz-Tomographie (ICMREIT) auf der Basis von monopolaren Schichtselektionsgradientenpulsen, die mittels einer Schichtselektionsgradientenspule angewendet werden, bei dem zwei getrennte Abtastungen einer abzubildenden Schicht durchgeführt werden, wobei ein MR-Phasenbild in jeder dieser Abtastungen mittels einer Anwendung einer Pulssequenz erhalten wird und wobei die zwei erhaltenen MR-Phasenbilder unterschiedlich sind, wobei

• wobei ein Strom in einem abzubildenden Objekt in den genannten zwei getrennten Abtastungen mittels einer Schichtselektionsgradientenwellenform mit positiver Polarität, SS-$G_z^+$, in der ersten Abtastung und einer Schichtselektionsgradientenwellenform mit negativer Polarität, SS-$G_z^-$, in der zweiten Abtastung induziert wird, die genannten SS-$G_z^+$ oder SS-$G_z^-$ Schichtselektionsgradientenwellenformen bestehend aus trapezförmigen Gradientenpulsen mit gleichen Amplituden und gegensätzlichen Polaritäten in Bezug zueinander,
• wobei die abzubildende Schicht an der Bedienerkonsole des MR-Abtasters in Bezug auf eine Lokalisierungsschicht, Loc, ausgewählt wird, als
• Physikalische Schicht, PhySlc, wenn SS-$G_z^+$ in der Pulssequenz verwendet wird, und als
• Symmetrische Schicht, SymSlc, wenn SS-$G_z^-$ in der Pulssequenz verwendet wird, so dass SymSlc in Bezug auf Loc symmetrisch zu PhySlc gewählt wird,
• die genannten PhySlc und SymSlc in der xy-Ebene liegen und der Abstand von PhySlc und SymSlc zu Loc entlang der z-Achse gleich ist, so dass in beiden Fällen die abzubildende Schicht der PhySlc entspricht,

wobei das Verfahren folgende Verfahrensschritte umfasst;

• eine Anregung der Spins mit einem selektiven und weichen Hochfrequenzpuls $S_x\alpha°$, der entlang der x-Achse angelegt wird, nachdem die abzubildende Schicht an der Bedienerkonsole des MR-Abtasters ausgewählt wurde,

• Bei Verwendung von SS-$G_z^+$ wird gleichzeitig mit $S_x\alpha°$ ein Gradient mit positiver Polarität zur Auswahl der Schicht des $S_x\alpha°$ Pulses, SS-$G_z^+[S_x\alpha°]$, angelegt, um eine selektive Anregung der Schicht zu erreichen, die mit dem $S_x\alpha°$ RF-Puls abzubilden ist, wobei die Amplitude von SS-$G_z^+[S_x\alpha°]$ gemäß der Schichtdicke bestimmt wird, gefolgt von einer Anwendung eines Phasenumkehrgradienten PhR-$G_z^+[S_x\alpha°]$, um den durch SS-$G_z^+[S_x\alpha°]$ erzeugten Phaseneffekt zu reduzieren,

• Bei Verwendung von SS-Gz- wird gleichzeitig mit $S_x\alpha°$ ein Gradient mit negativer Polarität zur Auswahl der Schicht des $S_x\alpha°$ Pulses, SS-$G_z^-[S_x\alpha°]$, angelegt, um eine selektive Anregung der Schicht zu erreichen, die mit dem $S_x\alpha°$ RF-Puls abzubilden ist, wobei die Amplitude von SS-$G_z^-[S_x\alpha°]$ gemäß der Schichtdicke bestimmt wird, gefolgt von einer Anwendung eines Phasenumkehrgradienten PhR-$G_z^-[S_x\alpha°]$, um den durch SS-$G_z^-[S_x\alpha°]$ erzeugten Phaseneffekt zu reduzieren,

• Nachfolgend der Anwendung der Gradientenpulse für die Schichtauswahl und die Phasenumkehr, eine Anwendung von phasencodierenden Gradientenpulsen entlang der x-Achse, $G_x$-p, und entlang der y-Achse, $G_y$-p, sofort nach den PhR-$G_z^+[S_x\alpha°]$ oder PhR-$G_z^-[S_x\alpha°]$ Pulsen,

• Nachfolgend der Anwendung der $G_y$-p und $G_x$-p Pulse eine Anwendung von strominduzierenden Gradientenpulsen mit positiver Polarität entlang der z-Achse, IC-$G_z^+$, um niederfrequenten (LF) Wirbelstrom $J_{LF}^+$ in dem abgebildeten Objekt zu induzieren, wenn SS-$G_z^+$ verwendet wird, und eine Anwendung von strominduzierenden Gradientenpulsen mit negativer Polarität entlang der z-Achse, IC-$G_z^-$, um LF-Wirbelstrom $J_{LF}^-$ in dem abgebildeten Objekt zu induzieren, wenn SS-$G_z^-$ verwendet wird, wobei das Intervall zwischen dem $S_x\alpha°$ Puls und dem Mittelpunkt des Plateau-Intervalls des jeweiligen ersten IC-$G_z^+$ oder IC-$G_z^-$ Pulses $t_B$ ist, und wobei das Intervall zwischen zwei benachbarten IC-$G_z^+$ oder IC-$G_z^-$ Pulsen 2*tc ist, wobei die Polaritäten und Amplituden der IC-$G_z^+$ oder IC-$G_z^-$ Pulse gleich den Polaritäten und den Amplituden von SS-$G_z^+[S_x\alpha°]$ und SS-$G_z^-[S_x\alpha°]$ jeweils sind,

• eine Anwendung von selektiven und weichen 180° Pulsen in y-Richtung, $S_y180°$, wobei der Mittelpunkt dieser Pulse mit den Mittelpunkten der Plateau-Intervalle der IC-$G_z^+$ und IC-$G_z^-$ Pulse zusammenfällt, um den MR-Bildern eine auf $J_{LF}^+$ oder $J_{LF}^-$bezogene Phasenkomponente hinzuzufügen,

• Nachfolgend der jeden $S_y180°$ Puls, mit Ausnahme des letzten Sy180° Pulses, eine Anwendung von nicht-selektiven, harten 180° Pulsen in y-Richtung, $H_y180°$, nach einem Intervall von tc im Anschluss an jeden der $S_y180°$ Pulse, um eine Phasenkomponente in Bezug auf $J_{LF}^+$ oder $J_{LF}^-$ zu den MR-Bildern hinzuzufügen, wobei die Gesamtzahl der $S_y180°$ und $H_y180°$ Pulse in der Pulssequenz ungerade ist, um ein messbares Echosignal zu erhalten,

• Nachfolgend der vorgenannten Pulse erfolgt eine Messung des Spinechosignals, das im Zeitintervall $t_B$ nach dem letzten $S_y180°$ Puls gebildet wird, mit gleichzeitiger Anwendung eines Datenerfassungsgradienten entlang der x-Achse symmetrisch in Bezug auf die Spinechobildungszeit,

• Erhalten der zwei verschiedenen MR-Phasenbilder durch Verwendung von SS-$G_z^+$ oder SS-$G_z^-$ in den zwei verschiedenen Abtastungen, aus den jeweiligen Messungen des Spinechosignals,

• Benennung der MR-Phasenbilder, die bei Abtastungen mit SS-$G_z^+$ oder SS-$G_z^-$erhalten wurden, jeweils als $\Phi^+$ und $\Phi^-$,

• Erhalten der niederfrequenten MR-Phasenverteilung $\Phi_{LF}$ als

$$\Phi_{LF} = \frac{\Phi^+ + \Phi^-}{2}$$

• Erhalten der Leitfähigkeitsverteilung $\sigma$ innerhalb von PhySlc als

$$\sigma = \frac{\left(\frac{\partial J_y}{\partial x} - \frac{\partial J_x}{\partial y}\right)}{\omega B_p},$$

Wobei Jx und Jy erhalten werden als

$$J_x = \frac{\partial \Phi_{LF}}{\partial y} \quad \text{und} \quad J_y = -\frac{\partial \Phi_{LF}}{\partial x}$$

Wobei $B_p$ der Wert der primären magnetischen Flussdichte der Gradientenspule bei PhySlc ist und unter Verwendung der Amplitude $G_z$ von SS-$G_z^+[S_x\alpha°]$ oder SS-$G_z^-[S_x\alpha°]$ und dem Abstand $d_z$ zwischen PhysSlc und Loc entlang der z-Achse berechnet wird als:

$$B_p = G_z d_z$$

wobei $\omega$ erhalten wird als

$$\omega = 2\pi/(4t_r)$$

wobei $t_r$ den Anstiegs- und Abfallzeiten der IC-$G_z^+$ oder IC-$G_z^-$ Pulse entspricht.

## Revendications

1. Procédé d'application d'une Tomographie d'Impédance Electrique par Résonance Magnétique à Courant Induit (ICMREIT) Séquence d'Impulsions Basée sur des Impulsions de Gradient de Sélection de Tranche Monopolaire appliquées au moyen d'une bobine de gradient de sélection de tranche où deux balayages séparés d'une tranche devant être imagée sont effectués, dans lequel une image de phase MR est obtenue dans chacun de ces balayages au moyen d'une application d'une séquence d'impulsions et dans lequel les deux images de phase MR obtenues sont différentes, dans lequel

     • un courant est induit dans un objet devant être imagé dans lesdits deux balayages séparés au moyen d'une forme d'onde de gradient de sélection de tranche avec une polarité positive, SS-$G_z^+$ dans le premier balayage, et une forme d'onde de gradient de sélection de tranche avec une polarité négative, SS-$G_z^-$ dans le second balayage, lesdites formes d'onde de gradient de sélection de tranche SS-$G_z^+$ ou SS-$G_z^-$ consistant en des impulsions de gradient trapézoïdales ayant des amplitudes égales et des polarités opposées les unes par rapport aux autres,
     • dans lequel la tranche devant être imagée est sélectionnée sur la console d'opérateur du scanner MR par rapport à une tranche de localisation, Loc, comme
     • Tranche physique, PhySlc, lorsque SS-$G_z^+$ est utilisé dans la séquence d'impulsion, et comme
     • Tranche symétrique, SymSlc lorsque SS-$G_z^-$ est utilisé dans la séquence d'impulsions, de sorte que SymSlc est choisi symétriquement à PhySlc par rapport à Loc,
     • lesdits PhySlc et SymSlc étant dans le plan xy et la distance de PhySlc et SymSlc à Loc étant égale le long de l'axe z, de sorte que dans les deux cas, la tranche devant être imagée correspond à PhySlc,

dans lequel le procédé comprend les étapes de procédure suivantes ;

     • une excitation des spins avec une impulsion radiofréquence (RF) sélective et douce $S_x\alpha°$, appliquée le long de l'axe x après que la tranche devant être imagée a été sélectionnée sur la console de l'opérateur du scanner MR,

     • Lorsque SS-$G_z^+$ est utilisé, une application d'un gradient de sélection de tranche de polarité positive de l'impulsion $S_x\alpha°$, SS-$G_z^+[S_x\alpha°]$, simultanément avec $S_x\alpha°$ afin de fournir une excitation sélective de la tranche devant être imagée avec l'impulsion RF $S_x\alpha°$, dans lequel l'amplitude de SS-$G_z^+[S_x\alpha°]$ est déterminée en fonction de l'épaisseur de la tranche, suivie par une application d'un gradient de réenroulement de phase PhR-$G_z^+[S_x\alpha°]$ afin de réduire l'effet de phase créé par SS-$G_z^+[S_x\alpha°]$,
     • Lorsque SS-$G_z^-$ est utilisé, une application d'un gradient de sélection de tranche de polarité négative de l'impulsion $S_x\alpha°$, SS-$G_z^-[S_x\alpha°]$ simultanément avec $S_x\alpha°$ afin de fournir une excitation sélective de la tranche devant être imagée avec l'impulsion RF $S_x\alpha°$,

dans lequel l'amplitude de SS-$G_z^-[S_x\alpha°]$ est déterminée en fonction de l'épaisseur de la tranche, suivie par une application d'un gradient de réenroulement de phase PhR-$G_z^-[S_x\alpha°]$ afin de réduire l'effet de phase créé par SS-$G_z^-[S_x\alpha°]$,

     • Après l'application des impulsions de gradient de sélection de tranche et de réenroulement de phase, une application d'impulsions de gradient de codage de phase le long de l'axe x, $G_x$-p, et le long de l'axe y, $G_y$-p, immédiatement après les impulsions PhR-$G_z^+[S_x\alpha°]$ ou PhR-$G_z^-[S_x\alpha°]$,
     • Après l'application des impulsions $G_y$-p et $G_x$-p, une application d'impulsions de gradient d'induction de courant avec une polarité positive le long de l'axe z, IC-$G_z^+$, afin d'induire un courant de Foucault de basse fréquence (LF) $J_{LF}^+$ dans l'objet imagé lorsque SS-$G_z^+$ est utilisé et une application d'impulsions de gradient d'induction de courant avec une polarité négative le long de l'axe z, IC-$G_z^-$, afin d'induire un courant de Foucault LF $J_{LF}^-$ dans l'objet imagé lorsque SS-$G_z^-$ est utilisé, dans lequel l'intervalle entre l'impulsion $S_x\alpha°$ et le point central de l'intervalle de plateau de la première impulsion IC-$G_z^+$ ou IC-$G_z^-$ respective est $t_B$ , et dans lequel l'intervalle entre deux impulsions IC-$G_z^+$ ou IC-$G_z^-$ adjacentes est $2*tc$ , dans lequel les polarités et les amplitudes desdites impulsions IC-$G_z^+$ ou IC-$G_z^-$ sont égales aux polarités et aux amplitudes SS-$G_z^+[S_x\alpha°]$ et SS-$G_z^-[S_x\alpha°]$ respectivement,
     • une application d'impulsions sélectives et douces de 180° dans la direction y, $S_y180°$, le centre desdites impulsions coïncidant avec les points centraux des intervalles de plateau des impulsions IC-$G_z^+$ et IC-$G_z^-$, afin d'ajouter aux images MR une composante de phase relative à $J_{LF}^+$ ou $J_{LF}^-$,

- Après chaque impulsion $S_y180°$ à l'exception de la dernière impulsion $S_y180°$, une application d'impulsions non sélectives et dures de 180° dans la direction y, $H_y180°$, après un intervalle de $t_C$ suivant chacune des impulsions $S_y180°$, afin d'ajouter une composante de phase relative à $J_{LF}^+$ ou $J_{LF}^-$ aux images MR, dans lequel le nombre total d'impulsions de $S_y180°$ et $H_y180°$ dans la séquence d'impulsions est impair afin d'obtenir un signal d'écho mesurable,

- Après les impulsions susmentionnées, une mesure du signal d'écho de spin formé à l'intervalle de temps $t_B$ après la dernière impulsion $S_y180°$, avec l'application simultanée d'un gradient d'acquisition de données le long de l'axe x symétriquement par rapport au temps de formation de l'écho de spin,

- Obtention desdites deux images de phase MR différentes en utilisant SS-$G_z^+$ ou SS-$G_z^-$dans lesdits deux balayages différents, à partir des mesures respectives du signal d'écho de spin,

- Nommage desdites images de phase MR obtenues dans les scans avec SS-$G_z^+$ ou SS-$G_z^-$ comme $\Phi^+$ et $\Phi^-$ respectivement,

- Obtention de la distribution de phase MR basse fréquence $\Phi_{LF}$ comme

$$\Phi_{LF} = \frac{\Phi^+ + \Phi^-}{2}$$

- Obtention de la distribution de la conductivité $\sigma$ à l'intérieur de PhySlc comme

$$\sigma = \frac{\left(\frac{\partial J_y}{\partial x} - \frac{\partial J_x}{\partial y}\right)}{\omega \mathrm{B}_p},$$

Dans lequel Jx et Jy sont obtenus comme

$$J_x = \frac{\partial \Phi_{LF}}{\partial y} \quad \text{et} \quad J_y = -\frac{\partial \Phi_{LF}}{\partial x}$$

Dans lequel $B_p$ est la valeur du flux magnétique primaire de la bobine de gradient de sélection de tranche à PhySlc et est calculée en utilisant l'amplitude, $G_z$ de SS-$G_z^+[S_x\alpha°]$ ou SS-$G_z^-[S_x\alpha°]$ et la distance $d_z$ entre PhysSlc et Loc le long de l'axe z comme :

$$\mathrm{B}_p = G_z d_z$$

dans lequel $\omega$ est obtenu comme

$$\omega = 2\pi/(4\mathrm{t}_r)$$

dans lequel $t_r$ correspond aux temps de montée et de descente des impulsions de IC-$G_z^+$ ou IC-$G_z^-$.

**Figure 1**

Figure 2

## REFERENCES CITED IN THE DESCRIPTION

### Patent documents cited in the description

- US 6397095 B1
- US 4978919 A
- WO 2013057655 A1
- WO 160494 A

### Non-patent literature cited in the description

- **ÖZPARLAK ; İDER.** Induced Current Magnetic Resonance Electrical Impedance Tomography. *Physiol. Meas.,* 2005, vol. 26 (2), 289-305
- Design and Implementation of Labview Based Data Acquisition and Image Reconstruction Environment for METU-MRI System. **ÖZSÜT.** Master of Science thesis. Graduate School of Natural and Applied Sciences, Department of Electrical and Electronic Engineering, ODTÜ, 2005
- **MANDIJA.** A Geometrical Shift Results in Erroneous Appearance of Low Frequency Tissue Eddy Induced Current Phase Maps. *Magn. Res. Med.,* 2016, vol. 76 (3), 905-912
- **ORAN.** Feasibility of Conductivity Imaging Using Subject Eddy Currents Induced by Switching of MRI Gradients. *Magn. Res., Med.,* 2016, vol. 77 (5), 1926-1937
- **GIBBS, LIU.** Feasibility of Imaging Tissue Electrical Conductivity by Switching Field Gradients with MRI. *Tomography,* 2015, vol. 1 (2), 125-135
- **H. H. EROQLU ; B. M. EYÜBOĞLU.** Induced current magnetic resonance electrical impedance tomography with z-gradient coils. *Proc. IEEE Int. Conf. Eng. in Med. and Biol. (EMBC 2014), Chicago,* 2014, 1143-1146
- **F. ORAN ; Y. Z. İDER.** Feasibility of conductivity imaging using subject eddy currents induced by switching of MRI gradients. *Magn. Reson. Med.,* 2017, vol. 77 (5), 1926-1937